(19) Europäisches Patentamt
European Patent Office
Office européen des brevets

(11) Publication number: **0 525 846 A1**

(12) **EUROPEAN PATENT APPLICATION**

(21) Application number: **92201829.6**

(22) Date of filing: **20.06.92**

(51) Int. Cl.5: **C12N 1/14**, C12P 7/40, //(C12P7/40,C12R1:645)

The applicant has filed a statement in accordance with Rule 28 (4) EPC (issue of a sample only to an expert). Accession number(s) of the deposit(s):ATCC 74072, 74073, 74074, 74075, 74076, 74083.

(30) Priority: **28.06.91 US 723357**

(43) Date of publication of application: **03.02.93 Bulletin 93/05**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Bills, Gerald F.**
**402 Aldene Road**
**Roselle, NJ 07203(US)**
Inventor: **Diez, Maria Teresa**
**C/Olid, 4**
**E-28010 Madrid(ES)**
Inventor: **Omstead, Mary Nallin**
**634 Westfield Avenue**
**Westfield, NJ 07090(US)**
Inventor: **Pelaez, Fernando**
**C/Camino de Valderribas, 110**
**E-28038 Madrid(ES)**
Inventor: **Kong, Yu Lin**
**57 Bryant Avenue**
**Edison, NJ 08820(US)**

(74) Representative: **Thompson, John Dr. et al**
**Merck & Co., Inc. European Patent**
**Department Terlings Park Eastwick Road**
**Harlow, Essex CM20 2OR(GB)**

(54) **Sporomiella intermedia and processes therefrom.**

(57) This invention relates to strains of Sporormiella intermedia that are isolated from the dung of herbivorous animals from xeric environments and are useful in a fermentation process to form compounds of formula (I):

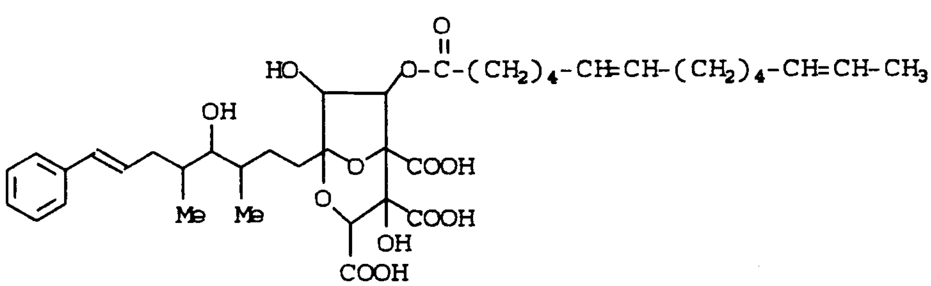

(I)

EP 0 525 846 A1

FIGURE I

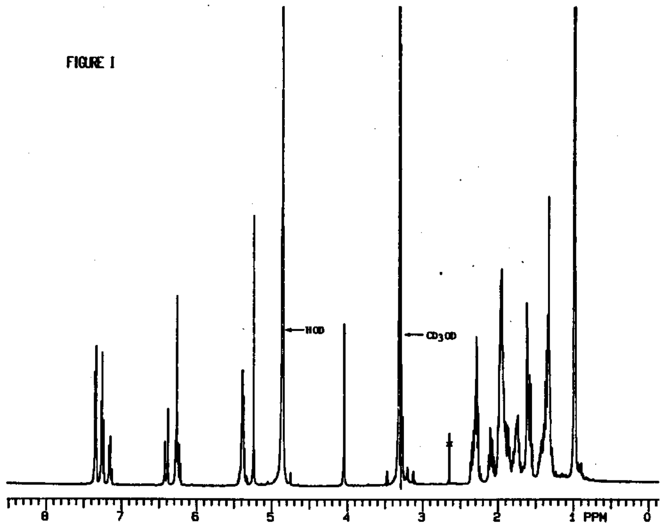

## BACKGROUND OF THE INVENTION

Hypercholesterolemia is known to be one of the prime risk factors for ischemic cardiovascular disease, such as arteriosclerosis. Bile acid sequestrants have been used to treat this condition; they seem to be moderately effective but they must be consumed in large quantities, i.e. several grams at a time and they are not very palatable.

MEVACOR® (lovastatin), now commercially available, is one of a group of very active antihypercholesterolemic agents that function by limiting cholesterol biosynthesis by inhibiting the enzyme, HMG-CoA reductase.

Squalene synthetase is the enzyme involved in the first committed step of the de novo cholesterol biosynthetic pathway. This enzyme catalyzes the reductive dimerization of two molecules of farnesyl pyrophosphate to form squalene. The inhibition of this committed step to cholesterol should leave unhindered biosynthetic pathways to ubiquinone, dolichol and isopentenyl t-RNA.

Previous efforts at inhibiting squalene synthetase have employed pyrophosphate or pyrophosphate analog containing compounds such as those described in P. Ortiz de Montellano et al, J. Med Chem. 20, 243 (1977) and E.J. Corey and R. Volante, J. Am. Chem. Soc. , 98, 1291 (1976). S. Biller (U.S. Patent 4,871,721) describes isoprenoid (phosphinylmethyl)phosphonates as inhibitors of squalene synthetase.

## SUMMARY OF THE INVENTION

The present invention provides strains of Sporormiella intermedia that produce nonphosphorous containing inhibitors of squalene synthetase.

## DETAILED DESCRIPTION OF THE INVENTION

The present invention is directed to novel microorganisms that produce compounds of structural formula (I) which are squalene synthetase inhibitors:

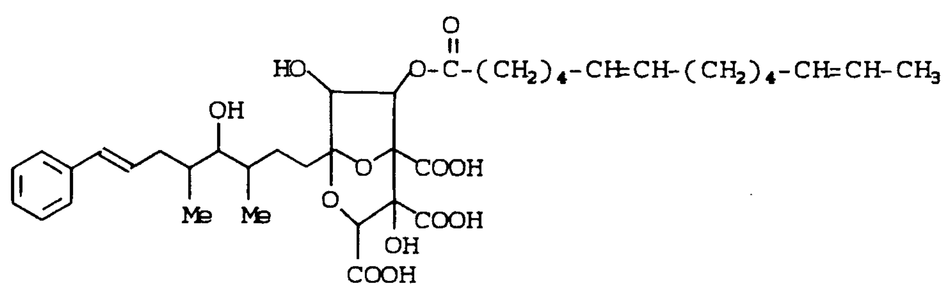

(I)

Compounds of formula (I) are useful as cholesterol-lowering agents and anti-fungal agents.

The compounds of formula (I) are prepared in an aerobic fermentation of Sporormiella intermedia. More particularly the strains employed may be selected from strains MF5651, MF5652, MF5655, MF5656, MF5657, MF5663 or a mutant organism thereof.

The culture MF5651 is that of a coprophilus fungus, Sporormiella intermedia, isolated from dung of elk (Arizona). This culture has been deposited with the American Type Culture Collection (ATCC) at 12301 Parklawn Drive, Rockville, MD 20852 as ATCC 74072. Culture MF5652 is that of a coprophilous fungus, Sporormiella intermedia, isolated from dung of giraffe (Kenya). This culture has been deposited as ATCC 74073. Culture MF5655 is that of a coprophilus fungus, Sporormiella intermedia, isolated from dung of goat (Greece). This culture has been deposited as ATCC 74074. Culture MF5656 is that of a coprophilous fungus, Sporormiella intermedia, isolated from dung of goat (Greece). This culture has been deposited as ATCC 74075. Culture MF5657 is that of a coprophilous fungus, Sporormiella intermedia, isolated from dung of goat (Greece). This culture has been deposited as ATCC 74076. Culture MF5663 is that of a coprophilous fungus, Sporormiella intermedia, isolated from dung of goat (Greece). This culture has been deposited as ATCC 74083.

In general, the Sporomiella intermedia strains of the present invention are coprophilous fungi. Many coprophilous fungi are specifically adapted to grow and disperse in dung and are unlikely to occur in other

substrata. Within the coprophilous fungal community, a distinct succession of species occurs over time, which contributes to the diversity of fungi recovered. Coprophilous fungi span a broad phylogenetic range. One of the most characteristic groups of coprophilous fungi are Ascomycetes variously classified in the genera Sporormia, Sporormiella, and Preussia. These saprobic fungi are particularly abundant on, but not necessarily restricted to, dung of herbivorous animals. These fungi are found with high frequency on dung in arid regions because the rate of decomposition of dung is slow and therefore there is a longer time for these coprophilous fungi to accumulate on the dung (S.I. Ahmed and R.F. Cain. 1972. Revision of the genera Sporormia and Sporormiella.. Canadian Journal of Botany. 50: 419-47; D.T. Wicklow. 1981. The coprophilous community: a mycological community for examining ecological ideas. pp. 47-76. In: The fungal community, its organization and role in the ecosystem. Eds. D.T. Wicklow & G.C. Carroll, Marcel Dekker, Inc.). These fungi also occur, but with much less frequency, as soil saprobes and as non-host-specific endophytes of woody plants.

The knowledge that these fungi frequently colonize dung of herbivores arid environments allows one to predictively isolate them into pure culture. This can be achieved by collecting dung in an arid region. Such a region might be dominated by vegetation of a desert, vegetation of the meditterranean-type, a desert scrubland, a grassland or a savannah. Dung can be incubated in a moist chamber at or near 100% relative humidity until ascomata develop, about 1-8 weeks. Mature ascospores can then be dissected from within the ascomata under a stereomicroscope with a fine needle and transferred to various standard mycological media to initiate a culture. Alternatively, these fungi can be obtained by collecting small pieces of the living stems of woody plants, surface sterilizing the stems, and applying the pieces to a standard yeast-malt extract agar containing antibacterial agents (Petrini, O. 1986. Taxonomy of endophytic fungi of aerial plant tissue. In: Microbiology of the Phyllosphere. Eds. N. J. Fokkema and J. van den Heuval, Cambridge Univ. Press).

A more direct approach to isolating these fungi is to pulverize a small amount of dried dung (0.5 - 2 g), serially dilute it in sterile water, and to then apply 0.1 - 1.0 mL of the suspension onto a selective mycological medium, such as DPY medium and CMC medium. A more selective approach is to filter the dung suspension through two wire meshes under running water. The pulverized dung is first washed through an upper mesh of 105 µm pore size; and then through a lower mesh of 90 µm. The particles are continually washed with water for 15-30 minutes. The particles that pass through the upper mesh but which are retained by the lower mesh are then dispersed onto the surface of a selective mycological medium, which is incubated until fungal colonies develop. Sporormiella colonies are generally recognized by their gray to black colors, velvety texture, and slow growth rate. Hyphal fragments of these initial colonies can then be transferred to a new agar medium to obtain a pure culture.

The composition of the selective mycological media are listed below:

DPY MEDIUM

| | |
|---|---|
| Dextrose | 5.0 g |
| Peptone | 1.0 g |
| Yeast extract | 2.0 g |
| $NH_4OH$ | 1.0 g |
| $K_2H_2PO_4$ | 1.0 g |
| $MgSO_4 \cdot 7H_2O$ | 0.5 g |
| $FeCl_3 \cdot 6H_2O$ | 0.5 mL of 1% (w/v) solution |
| Oxgall (dried bovine bile) | 5.0 g |
| Sodium propionate | 1.0 g |
| Agar | 20.0 g |
| Water | 1000 mL |
| Autoclave 20 min. (121 ° C, 15 psi) | |

After autoclaving add:

| | |
|---|---|
| Chlorotetracycline | 50.0 mg |
| Streptomycin sulfate | 50.0 mg |

(or any other appropriate antibacterial antibiotics)

CMC MEDIUM

| | |
|---|---|
| Sodium carboxymethyl-cellulose | 10.0 g |
| Yeast extract | 0.5 g |
| $(NH_4)_2SO_4$ | 1.0 g |
| $NaNO_3$ | 2.0 g |
| $KH_2PO_4$ | 1.0 g |
| $MgSO_4 \cdot 7H_2O$ | 0.5 g |
| KCl | 0.5 g |
| $CaCl_2$ | 0.05 g |
| $FeSO_4 \cdot 7H_2O$ | 0.01 g |
| $CuSO_4$ | 0.01 g |
| $MnSO_4 \cdot 4H_2O$ | 0.005 g |
| $ZnSO_4 \cdot 7H_2O$ | 0.001 g |
| Agar | 15.0 g |
| Water | 1000 mL |
| Autoclave 20 min. (121°C, 15 psi) | |

After autoclaving, add:

| | |
|---|---|
| Chlorotetracycline | 50.0 mg |
| Streptomycin sulfate | 50.0 mg |

(or any other appropriate antibacterial antibiotics)

Strains of Sporormiella intermedia exhibit the following morphological features.

Colonies relatively slow-growing, in 2 weeks attaining a diameter of: 10-15 mm on cornmeal agar (Difco Laboratories); 10-15 mm on oatmeal agar (Difco Laboratories); 15-20 mm on V8 juice agar (200 ml V8 juice, Campbell Soup Co., 3 g Ca $CO_3$, 20 g agar diluted to 1 L distilled water). On oatmeal agar slightly raised, about 0.5-1 mm deep, with submerged margin, with surface felty to velutinous, cream when young, soon pale gray to dark gray, or finally dark olivaceous gray to almost black, Cartridge Buff (capitalized color names from Ridgway, R, Color Standards and Nomenclature, Washington, D.C. 1912), Marguerite Yellow, Olive, Buff, Light Grayish Olive, Grayish Olive, Deep Grayish Olive, Iron Gray, Olivaceous Black. In reverse, dull yellowish olive to olivaceous gray to dark olivaceous gray. Odors and exudates absent. Often extensive dull to shiny, black stromatic regions develop in colonies after 2-3 weeks. Stromatic regions may contain many embedded, confluent to gregarious pseudothecia initials. Colonies of many strains have a strong tendency to develop aberrent and attenuated sectors, especially after repeated tranfer. These sectors are generally paler in color and have lost or have reduced their ability to differentiate stromata and/or pseudothecia.

Ascoma a pseudothecium. Pseudothecia evident in 2-3 weeks, maturing in 4-5 weeks on oatmeal agar at 25°C in continuous fluorescent light. Pseudothecia single to densely gregarious, embedded, with upper 10-50% protruding above the surface, 200-300 µm in diameter, globose to subglobose, with or without an apical papilla, non-ostiolate, glabrous, dull, uniformly black. In culture, pseudothecia often become moribund and fail to fully mature within 4-8 weeks. Often development is arrested with only the formation of asci initials and paraphyses. Peridium thin, 1-2 cells thick, a textura angularis. Peridial cells isodiametric, 4-8 µm in diameter, gray to dark olivaceous gray in KOH. Asci abundant, arising from a common basal area, bitunicate, 8-spored, cylindrical, straight to slightly curved, with broad rounded apex, 120-180 x 20-35 µm with a distinct basal stalk, with basal stalk 7-11 µm long. Paraphyses abundant, intermixed with asci, filamentous, 1-3 µm wide, septate, approximately equal in length with asci. Ascospores biseriate within the ascus, 42-58 x 10-12 µm, 4-celled, deeply constricted at the septa, terminal cells with rounded or tapered apices, central cells oblong to doliform, each cell with an obscure lateral germ slit, with entire ascospore surrounded by a thin, refractive, hyaline sheath, with cells often easily separating, dark olivaceous gray in KOH.

A biologically pure culture of Sporomiella intermedia as claimed herein is defined as being isolated

from the natural environment and free of viable contaminating microorganisms. A culture of Sporormiella intermedia as claimed herein is defined as being isolated from the natural environment and free of viable contaminating microorganisms that would be deleterious to the formation of a compound of formula (I). An active mutant of a culture as described herein is defined as a culture having one or more mutations and capable of producing compounds of formula (I).

Although in general the formation of a particular secondary metabolite is unpredictable, Applicants have surprisingly found that strains of Sporormiella intermedia are chemotaxonomically similar in their ability to produce inhibitors of squalene synthetase and compounds of formula (I). These strains are readily detected by a rapid two-stage screening procedure. Strains are isolated and cultivated in liquid nutrient media. Small samples are removed periodically and the final fermentation broth is frozen and stored. The small samples are used in the first stage of the screening procedure. These samples are extracted with an organic solvent. The organic phase is concentrated and tested in the squalene synthetase assay described below. The presence of a compound that inhibits the activity of squalene synthetase is determined by comparing the squalene synthetase activity of the sample to the squalene synthetase activity of the control. Fermentation broths which give a positive result in the squalene synthetase assay are tested in the second stage of the screening procedure. The frozen fermentation broth is thawed and extracted with an organic solvent. The organic phase is concentrated and analyzed by high performance liquid chromatography (HPLC). The presence of compounds of formula (I) is determined by comparing the chromatogram of the extracted sample to the chromatogram of authentic (I). Confirmation of the presence of (I) in the extract is obtained from the UV spectrum of the isolated component and from MS data of the isolated components and derivatives thereof.

The culture MF5651 has been identified as Sporormiella intermedia and exhibits all the essential morphological features of that species as described in the general description presented above and in the description of S.I. Ahmed and R.F. Cain. 1972. Revision of the genera Sporormia and Sporormiella. Canadian Journal of Botany. 50: 419-477. These diagnostic morphological features include: relatively slow-growing, pale gray, dark gray to black colonies on standard mycological media such as potato-dextrose, oatmeal, or malt-yeast extract agar, absence of a conidial state in cultures; formation of black stromatic tissues and/or pseudothecia on the colony surface after 2-5 weeks; formation of pseudothecia that are globose to subglobose, glabrous, without an ostiole, or faintly ostiolate; filamentous paraphyses formed among the asci within the pseudothecial cavity; asci that are bitunicate, cylindrical, with a short stem-like base; and ascospores that are biseriately arranged within the ascus, with dimensions between 45-60 $\mu$m long and 10-12 $\mu$m wide, four-celled, constricted at the septa, surrounded by a thin, gelatinous sheath pigmented olive-gray, with each cell having a lateral, curved germslit.

The culture MF5652 has been identified as Sporormiella intermedia and exhibits all the essential morphological features of that species as described in the general description presented above and in the description of S.I. Ahmed and R.F. Cain. 1972. Revision of the genera Sporormia and Sporormiella. Canadian Journal of Botany. 50: 419-477. These diagnostic morphological features include: relatively slow-growing, pale gray, dark gray to black colonies on standard mycological media such as potato-dextrose, oatmeal, or malt-yeast extract agar, absence of a conidial state in cultures; formation of black stromatic tissues and/or pseudothecia on the colony surface after 2-5 weeks; formation of pseudothecia that are globose to subglobose, glabrous, without an ostiole, or faintly ostiolate; filamentous paraphyses formed among the asci within the pseudothecial cavity; asci that are bitunicate, cylindrical, with a short stem-like base; and ascospores that are biseriately arranged within the ascus, with dimensions between 45-60 $\mu$m long and 10-12 $\mu$m wide, four-celled, constricted at the septa, surrounded by a thin, gelatinous sheath pigmented olive-gray, with each cell having a lateral, curved germslit.

Cultures MF5655, MF5656, MF5657 and MF5663 were isolated from the same enviroment. Cultures MF5655, MF5656 and MF5657 have been identified as Sporormiella intermedia and exhibit all of the essential morphological features of that species as described in the general description above and in the description of S.I. Ahmed and R.F. Cain. 1972. Revision of the genera Sporormia and Sporormiella. Canadian Journal of Botany. 50: 419-477. These diagnostic morphological features include: relatively slow-growing, pale gray, dark gray to black colonies on standard mycological media such as potato-dextrose, oatmeal, or malt-yeast extract agar, absence of a conidial state in cultures; formation of black stromatic tissues and/or pseudothecia on the colony surface after 2-5 weeks; formation of pseudothecia that are globose to subglobose, glabrous, without an ostiole, or faintly ostiolate; filamentous paraphyses formed among the asci within the pseudothecial cavity; asci that are bitunicate, cylindrical, with a short stem-like base; and ascospores that are biseriately arranged within the ascus, with dimensions between 45-60 $\mu$m long and 10-12 $\mu$m wide, four-celled, constricted at the septa, surrounded by a thin, gelatinous sheath pigmented olive-gray, with each cell having a lateral, curved germslit.

Compounds of formula (I) can be obtained by culturing an above noted microorganism in an aqueous nutrient medium containing sources of assimilable carbon and nitrogen, preferably under aerobic conditions. Nutrient media may also contain mineral salts and defoaming agents.

The preferred sources of carbon in the nutrient medium are carbohydrates such as glucose, glycerin, starch, dextrin, and the like. Other sources which may be included are maltose, mannose, sucrose, and the like. In addition, complex nutrient sources such as oat flour, corn meal, millet, corn and the like may supply utilizable carbon. The exact quantity of the carbon source which is used in the medium will depend, in part, upon the other ingredients in the medium, but is usually found in an amount ranging between 0.5 and 5 percent by weight. These carbon sources can be used individually in a given medium or several sources in combination in the same medium.

The preferred sources of nitrogen are amino acids such as glycine, methionine, proline, threonine and the like, as well as complex sources such as yeast extracts (hydrolysates, autolysates), dried yeast, tomato paste, soybean meal, peptone, corn steep liquor, distillers solubles, malt extracts and the like. Inorganic nitrogen sources such as ammonium salts (e.g., ammonium nitrate, ammonium sulfate, ammonium phosphate, etc.) can also be used. The various sources of nitrogen can be used alone or in combination in amounts ranging between 0.2 to 90 percent by weight of the medium.

The carbon and nitrogen sources are generally employed in combination, but need not be in pure form. Less pure materials which contain traces of growth factors, vitamins, and mineral nutrients may also be used. Mineral salts may also be added to the medium such as (but not limited to) calcium carbonate, sodium or potassium phosphate, sodium or potassium chloride, magnesium salts, copper salts, cobalt salts and the like. Also included are trace metals such as manganese, iron, molybdenum, zinc, and the like. In addition, if necessary, a defoaming agent such as polyethylene glycol or silicone may be added, especially if the culture medium foams seriously.

The preferred process for production of compounds of this invention consists of inoculating spores or mycelia of the producing organism into a suitable medium and then cultivating under aerobic condition.

The fermentation procedure generally is to first inoculate a preserved source of culture into a nutrient seed medium and to obtain, sometimes through a two step process, growth of the organisms which serve as seeds in the production of the active compounds. After inoculation, the flasks are incubated with agitation at temperatures ranging from 20 to 30° C, preferably 25 to 28° C. Agitation rates may range up to 400 rpm, preferably 200 to 220 rpm. Seed flasks are incubated over a period of 2 to 10 days, preferably 2 to 4 days. When growth is plentiful, usually 2 to 4 days, the culture may be used to inoculate production medium flasks. A second stage seed growth may be employed, particularly when going into larger vessels. When this is done, a portion of the culture growth is used to inoculate a second seed flask incubated under similar conditions but employing shorter time.

After inoculation, the fermentation production medium is incubated for 5 to 25 days, preferably 7 to 22 days, with or without agitation (depending on whether liquid or solid fermentation media are employed). The fermentation is conducted aerobically at temperatures ranging from 20 to 40° C. If used, agitation may be at a rate of 200 to 400 rpm. To obtain optimum results, the temperatures are in the range of 22 to 28° C, most preferably 24 to 26° C. The pH of the nutrient medium suitable for producing the active compounds is in the range of 3.5 to 8.5, most preferably 4.0 to 7.0. After the appropriate period for production of the desired compound, fermentation flasks are harvested and the active compound isolated.

An alcoholic solvent, optionally mixed with a non-alcoholic, oxygenated solvent, such as an ester or a ketone, is employed to extract a compound of this invention from a solid fermentation medium.

The mixture is vigorously stirred and filtered, and the filtrate is concentrated under reduced pressure. Water is added to the concentrate and the pH is adjusted with a mineral acid to between 1 and 4, most preferably between pH 1.5 and 2.5. The aqueous concentrate is then repeatedly extracted with a water immiscible, oxygenated solvent. The water immiscible organic layer is removed and evaporated to dryness. The residue is then generally subjected to several separation steps such as adsorption and partition chromatography, and precipitation. For each separation step, fractions are collected and combined based on results from a biological assay and/or HPLC/TLC analysis.

The preferred solvent for extraction of the solid fermentation is a 1:1 mixture of methanol and 2-butanone. After concentrating the initial extract and diluting with water, the preferred partitioning solvent is dichloromethane or ethyl acetate.

For extraction of compound (I) from a liquid fermentation, an alcoholic solvent or a non-alcoholic oxygenated solvent can be used. A preferred alcoholic solvent is methanol, in which case the liquid fermentation is treated with two to four volumes of methanol and is then stirred vigorously. The mixture is then filtered and the filtrate is concentrated under reduced pressure. Water is added to the concentrate, the pH is adjusted with mineral acid to between 1 and 4, most preferrably between pH 1.5 and 2.5. The

aqueous concentrate is then extracted repeatedly with a water immiscible oxygenated solvent or chlorohydrocarbon solvent. The water immiscible organic layer is decanted and concentrated to dryness. The residue is then further purified as described above for the evaporated organic extract from solid fermentations.

A preferred oxygenated solvent for extraction of liquid fermentations is ethyl acetate. The liquid fermentation is first adjusted with mineral acid to between pH 1 and 4, most preferably between pH 1.5 and 2.5. The mixture is then extracted repeatedly with an oxygenated solvent such as ethyl acetate or 2-butanone. The water immiscible organic layer is decanted and concentrated to dryness. The residue is then further purified as described above for the evaporated organic extract from solid fermentations. Liquid fermentations can also be extracted with 2-butanone without acidification of broth. In this case, the organic extract is concentrated to an aqueous layer, acidified to pH 1.5-2.5 and extracted with an oxygenated solvent such as ethyl acetate.

The chromatographic separations may be carried out by employing conventional column chromatography with ionic or nonionic resin. Silica gel, such as that available from E. Merck, is a useful adsorbent. When silica gel is the adsorbent, an alcohol/chlorohydrocarbon/organic acid mixture such as methanol/chloroform/acetic acid/ water is useful as an eluant. For reverse phase chromatography, the preferred adsorbent is a C8 bonded phase silica gel, although bonded phase silica gels with longer or shorter alkyl residues are also useful, as are hydrophobic resins with a polystyrene/divinylbenzene backbone, such as Mitsubishi Dianion HP-20, or those with a polyacrylate backbone. The preferred eluant for reverse phase chromatography is a mixture of acetonitrile and water buffered at a low pH, such as 0.1% phosphoric acid, or trifluoroacetic acid. Ionic resins such as Dowex-1 ($Cl^-$) or Dowex-50 ($Ca^{++}$) are also useful in the purification. Of particular utility are anion exchange resins such as BioRAD AG4x4 (formate) and Amberlyst A21 (acetate). The active compound can be precipitated out of a nonpolar solvent as the quinine salt. The preferred solvent for precipitation is diethyl ether. The active compound (I) can also be precipitated out of polar solvents, such as methanol, as the ammonium salt.

The intrinsic squalene synthetase inhibitory activity of the compounds formed in the fermentation of the microorganisms of the present invention was measured by the standard in vitro protocol described below:

Preparation of Rat Liver Microsomes:

Male, Charles River CD rats (120 to 150 g) were fed a diet containing 0.1% lovastatin for 4 days. The livers from these rats were homogenized in 5 volumes (ml/g) of ice cold 50 mM HEPES (4-(2-hydroxyethyl)-1-piperazine-ethanesulfonic acid), 5 mM EDTA(ethylenediaminetetraacetic acid) pH 7.5 with a Potter-Elvehjem type tissue grinder. The homogenate was centrifuged twice at 20,000 x g for 15 minutes at 4°C, discarding the pellet each time. The supernatant was then centrifuged at 100,000 x g for 1 hour at 4°C. The resulting microsomal pellet was resuspended in a volume of the above homogenizing buffer equal to one-fifth the volume of the original homogenate. This microsomal preparation has a protein concentration of about 7 mg/mL. The microsomal suspensions were stored in aliquots at -70°C. Squalene synthetase activity in these aliquots is stable for at least several months.

Partial Purification of Prenyl Transferase

Prenyl transferase was purified to use in the enzymatic synthesis of radiolabelled farnesyl pyrophosphate. Prenyl transferase was assayed by the method of Rilling (Methods in Enzymology 110, 125-129 (1985)) and a unit of activity is defined as the amount of enzyme that will produce 1 μmole of farnesyl pyrophosphate per minute at 30°C in the standard assay.

The livers of 23 forty-day old male rats that had been fed 5% cholestyramine plus 0.1% lovastatin were homogenized in a Waring blender in 1 liter of 10 mM mercaptoethanol, 2 mM EDTA, 25 μM leupeptin, 0.005% phenylmethyl sulfonyl fluoride pH 7.0 containing 0.1 trypsin inhibitor units of aprotinin/ml. The homogenate was centrifuged at 20,000 x g for 20 minutes. The supernatant was adjusted to pH 5.5. with 6 N HOAc and centrifuged at 100,000 x g for 1 hour. This supernatant was adjusted to pH 7.0 with 3 N KOH and a 35-60% ammonium sulfate fraction taken. The 60% pellet was redissolved in 60 ml of 10 mM potassium phosphate, 10 mM mercaptoethanol, 1 mM EDTA pH 7.0 (Buffer A) and dialyzed against two 1 liter changes of Buffer A. This dialyzed fraction was applied to a 12.5 x 5 cm column of DEAE-sepharose 4B equilibrated with Buffer A. The column was washed with 700 mL of Buffer A and a 1 liter gradient from Buffer A to 100 mM potassium phosphate, 10 mM mercaptoethanol, 1 mM EDTA pH 7.0. Fractions having a specific activity greater than 0.20 units/mg were combined, solid ammonium sulfate was added to bring to 60% saturation and pelleted. The pellet was dissolved in 8 ml of 10 mM Tris, 10 mM β-mercaptoethanol pH

7.0 (Buffer B). The redissolved pellet was taken to 60% saturation with ammonium sulfate by adding 1.5 volumes of saturated ammonium sulfate in Buffer B. This ammonium sulfate suspension contained 3.5 units/mL with a specific activity of 0.23 units/mg and was free of isopentenyl pyrophosphate isomerase activity. This ammonium sulfate suspension was used for the synthesis of [4-$^{14}$C]farnesyl-pyrophosphate and its activity was stable stored at 4°C for at least 6 months.

Enzymatic Synthesis of [4-$^{14}$C]farnesyl-pyrophosphate

The solvent (ethanol: 0.15 N $NH_4OH$, 1:1) was removed from 55 µCi of [4-$^{14}$C]isopentenyl pyrophosphate(47.9 µCi/µmole) by rotary evaporation. Six hundred microliters of 100 mM Tris, 10 mM $MgCl_2$, 4 mM dithiothreitol pH 7.5 was added and the solution was transferred to a 1.5 ml Eppendorf centrifuge tube. Geranyl-pyrophosphate, 250 µl of a 20 mM solution, and 50 µl of the ammonium sulfate suspension of prenyl transferase were added to initiate the reaction. This incubation contained 5 µmoles of geranyl pyrophosphate, 1.15 µmoles of isopentenyl pyrophosphate, 6 µmoles of $MgCl_2$ of 0.18 units of prenyl transferase in a volume of 900 µl. The incubation was conducted at 37°C. During the incubation, the mix turned cloudy white as the newly formed magnesium complex of farnesyl pyrophoshate precipitated out of solution. The [4-$^{14}$C]farnesyl pyrophosphate was collected by centrifugation for 3 minutes at 14,000 rpm in an Eppendorf centrifuge tube, the supernatant removed, and the pellet was dissolved in 1.0 ml of 50 mM HEPES, 5 mM EDTA, pH 7.5 The yield was 50.7 µCi (92%) of [4-$^{14}$C]farnesyl pyrophosphate. The [4-$^{14}$C]-farnesyl pyrophosphate was stored in aliquots at -70°C.

Squalene Synthetase Assay

Reactions were performed in 16 x 125 mm screw cap test tubes. A batch assay mix was prepared from the following solution:

| | µl per assay | volume for 50 assays |
|---|---|---|
| 1. 250 mM HEPES pH 7.5 | 20 | 1000 |
| 2. NaF 110 mM | 10 | 500 |
| 3. $MgCl_2$ 55 mM | 10 | 500 |
| 4. Dithiothreitol 30 mM | 10 | 500 |
| 5. NADPH 10 mM (made fresh) | 10 | 500 |
| 6. [4-$^{14}$C]farnesyl-pyrophosphate 47.9 µCi/µmole, and 0.025 µCi/3.0 µl | 3.0 | 150 |
| 7. $H_2O$ | 24 | 1200 |

This assay mix was degassed under a vacuum and flushed with $N_2$. Solutions of the squalene synthetase inhibitors were prepared either in DMSO or MeOH and a 1:120 dilution of the microsomal protein was made with the original homogenizing buffer. For each reaction, 87 µl of the assay mix was taken with 3 µl of an inhibitor solution (DMSO or MeOH in the controls), warmed to 30°C in a water bath and then the reaction was initiated by the addition of 10 µl of the 1:120 dilution of microsomal protein (0.6 µg protein total in the assay). The reactions were stopped after 20 minutes by the addition of 100 µl of a 1:1 mix of 40% KOH with 95% EtOH. The stopped mix was heated at 65°C for 30 minutes, cooled, 10 mL of heptane was added and the mix was vortexed. Two g of activated alumina was then added, the mix vortexed again, the alumina allowed to settle and 5 mL of the heptane layer was removed. Ten ml of scintillation fluid was added to the heptane solution and radioactivity was determined by liquid scintillation counting.

Percent inhibition is calculated by the formula:

$$1 - \frac{[\text{Sample - Blank}]}{[\text{Control - Blank}]} \times 100$$

The following examples illustrate the use of the microorganisms herein for the preparation of the compounds of formula (I) and, as such, are not to be considered as limiting the invention set forth in the claims appended hereto.

The composition of media employed in the following Examples are listed below:

YME Plating Medium

| Component | Amount |
|---|---|
| Yeast Extract | 4.0 g |
| Malt Extract | 10.0 g |
| Glucose | 4.0 g |
| Distilled $H_2O$ | 1000 mL |
| Agar | 25.0 g |

KF SEED MEDIUM

| | per liter |
|---|---|
| Corn Steep Liquor | 5 g |
| Tomato Paste | 40 g |
| Oat Flour | 10 g |
| Glucose | 10 g |
| Trace Element Mix | 10 mL |

pH adjusted to 6.8 (presterile)
50 mL/nonbaffled 250 mL Erlenmeyer flask
autoclave 20 minutes (121°C, 15 psi)

Trace Element Mix

| | g/L |
|---|---|
| $FeSO_4 \bullet 7H_2O$ | 1.0 |
| $MnSO_4 \bullet 4H_2O$ | 1.0 |
| $CuCl_2 \bullet 2H_2O$ | 0.025 |
| $CaCl_2 \bullet 2H_2O$ | 0.1 |
| $H_3BO_3$ | 0.056 |
| $(NH_4)_6Mo_7O_{24} \bullet 4H_2O$ | 0.019 |
| $ZnSO_4 \bullet 7H_2O$ | 0.2 |

dissolved in 1 L 0.6 N HCl

Production Media

Solid Substrate Production Medium
(solid substrate media prepared in nonbaffled 250 mL Erlenmeyer flasks)

**F1**

| | |
|---|---|
| Cracked corn | 10.0 g/flask |
| Base liquid #1 | 10.0 mL/flask |

**F202**

| | |
|---|---|
| Millet | 15.0 g/flask |
| Base liquid #4 | 15.0 mL/flask |

Base Liquid #1

| | g/L |
|---|---|
| Ardamine PH | 0.2 |
| $KH_2PO_4$ | 0.1 |
| $MgSO_4 \bullet 7H_2O$ | 0.1 |
| Sodium tartrate | 0.1 |
| $FeSO_4 \bullet 7H_2O$ | 0.01 |
| $ZnSO_4 \bullet 7H_2O$ | 0.01 |
| distilled $H_2O$ | 1000 mL |

no pH adjustment
autoclave 15 minutes
(121°C, 15 psi)
add 15.0 mL distilled $H_2O$/flask
autoclave 20 minutes
(121°C, 15 psi)

Base Liquid #4

| | g/L |
|---|---|
| Yeast Extract | 33.3 |
| Sodium tartrate | 6.67 |
| Sucrose | 33.3 |
| Alfalfa | 33.3 |
| Corn Oil | 6.67 |
| $FeSO_4 \bullet 7H_2O$ | 0.67 |
| distilled $H_2O$ | 1000 mL |

no pH adjustment
autoclave 15 minutes
(121°C, 15 psi)
add 15.0 mL distilled $H_2O$/flask
autoclave 20 minutes
(121°C, 15 psi)

Liquid Production Media
[all liquid formulations dispensed 45 mL per nonbaffled 250 mL Erlenmeyer flask autoclaved for 20 minutes (121°C, 15 psi)]

**LSF1**

| | g/L |
|---|---|
| Glycerol | 75.0 |
| Glucose | 10.0 |
| Ardamine PH | 5.0 |
| Soybean meal | 5.0 |
| Tomato paste | 5.0 |
| Sodium citrate | 2.0 |
| $(NH_4)_2SO_4$ | 2.0 |
| distilled $H_2O$ | 1000 mL |

presterile pH adjusted to 7.0

**CYS 80**

| | g/L |
|---|---|
| Cornmeal (yellow) | 50.0 |
| Yeast extract | 1.0 |
| Sucrose | 80.0 |
| distilled $H_2O$ | 1000 mL |

no pH adjustment

EXAMPLE 1

Preparation of Compound I

A. Culturing Fungus MF5651

Culture MF5651 was inoculated into KF seed medium using one glass scoop of hyphal fragments preserved in sterile soil. The KF seed flask was incubated for 71 hours at 25°C, 220 rpm, 85% humidity. At the end of this incubation, 2.0 mL aliquots were aseptically transferred to each of 12 LSF1 production medium flasks. These production flasks were then incubated at 25°C, 220 rpm, 85% humidity for 7 days. Flasks were harvested by homogenizing the mycelial growth for 20 seconds at high speed using a Biohomogenizer/mixer (Biospec Products Inc., Bartlesville, OK). The whole broth contents of the flasks were then pooled into a 1 L Erlenmeyer flask.

B. Isolation of Compound I

A 50 mL aliquot of the fermentation of Example 1, Part A was adjusted to pH 2.5 with 2.5 N HCl and 50 mL ethyl acetate was added. The mixture was shaken 1 hour at room temperature and the layers were separated by centrifugation in an IEC clinical centrifuge. The ethyl acetate layer was removed and concentrated in vacuo. The residue was dissolved in 20 mL of a pH 4.3 solution of 40 mM sodium formate in 6/4 methanol/$H_2O$. The resulting solution was defatted by shaking briefly with an equal volume of hexane and the hexane layer was discarded.

The defatted aqueous methanol solution above was applied to a 3 mL column of BioRad AG4x4 (formate cycle, 100-200 mesh) at approximately 10 column volumes/hour. The column was washed with 25 mL of a pH 4.3 solution of 40 mM sodium formate in 6/4 methanol/$H_2O$ and then with 20 mL of 6/4 $CH_3CN/H_2O$. The column was eluted with 0.2 N $H_2SO_4$ in 6/4 $CH_3CN/H_2O$, collecting 10 mL fractions. Fractions 3 and 4 contained Compound I.

The AG4x4 eluates containing Compound I were pooled and 20 mL of 0.1 N HCl containing 10% (w/v) NaCl was added. This solution was then extracted once with 15 mL $CH_2Cl_2$; the $CH_2Cl_2$ layer was removed, dried over anhydrous $Na_2SO_4$ and concentrated in vacuo.

The resulting residue was dissolved in 2 mL methanol for analytical HPLC (Phenomenex Ultracarb 5 ODS30, 15 cm x 4.6 mm, 65% $CH_3CN/H_2O$ + 0.1% $H_3PO_4$ at 1.0 mL/min., room temperature, Waters 990+ diode array detection) and found to contain 27 µg/mL Compound, I $t_r$ = 15.8 minutes.

## EXAMPLE 2

### Preparation of Compound I

A. Culturing Fungus MF5652

Culture MF5652 was inoculated into KF seed medium using one glass scoop of hyphal fragments preserved in sterile soil. The KF seed flask was incubated for 72 at 25° C, 220 rpm, 85% humidity. At the end of this incubation, 2.0 mL aliquots were aseptically transferred to each of 12 F1 solid production medium flasks and 12 LSF1 liquid production medium flasks. These production flasks were then incubated at 25° C, 220 rpm, 85% humidity, either statically (F1 flasks) or on a gyrotory shaker at 220 rpm (LSF1 flasks) for 21 days. At harvest 50 mL of methyl ethyl ketone were added to each F1 production flask and the solid growth was manually broken apart into smaller pieces. Solvent-treated flasks were returned to the gyrotory shaker for agitation at 220 rpm for 30 min in order to further break apart the mycelial mass as well as to improve contact of the solvent with the cells. After shaking, the contents of these flasks were pooled by pouring the entire contents of the flasks (solids and all) into a 1 L flask. LSF1 flasks were harvested by homogenizing the mycelial growth for 20 seconds at high speed using a Biohomogenizer/mixer (Biospec Products Inc., Bartlesville, OK). The whole broth contents of the LSF1 flasks were then pooled into a 1 L Erlenmeyer flask.

B. Isolation of Compound I

A 50 mL aliquot of the LSF1 liquid fermentation of Example 2, Part A was adjusted to pH 2.5 with 2.5 N HCl and 50 mL ethyl acetate was added. The mixture was shaken 1 hour at room temperature and the layers were separated by centrifugation in an IEC clinical centrifuge. The ethyl acetate layer was removed and concentrated in vacuo. The residue was dissolved in 20 mL of a pH 4.3 solution of 40 mM sodium formate in 6/4 methanol/$H_2O$. The resulting solution was defatted by shaking briefly with an equal volume of hexane and the hexane layer was discarded.

The defatted aqueous methanol solution above was applied to a 3 mL column of BioRad AG4x4 (formate cycle, 100-200 mesh) at approximately 10 column volumes/hour. The column was washed with 25 mL of a pH 4.3 solution of 40 mM sodium formate in 6/4 methanol/$H_2O$ and then with 20 mL 6/4 $CH_3CN/H_2O$. The column was eluted with 0.2 N $H_2SO_4$ in 6/4 $CH_3CN/H_2O$, collecting 10 mL fractions. Eluates 3 and 4 contained Compound I.

The AG4x4 eluates containing Compound I were pooled and 20 mL of 0.1 N HCl containing 10% (w/v) NaCl was added. This solution was then extracted once with 15 mL $CH_2Cl_2$; the $CH_2Cl_2$ layer was removed, dried over anhydrous $Na_2SO_4$ and concentrated in vacuo. The $CH_2Cl_2$ layer was dissolved in 2 mL methanol for analytical HPLC (Phenomenex Ultracarb 5 ODS30, 15 cm x 4.6 mm, 65% $CH_3CN/H_2O$ + 0.1% $H_3PO_4$ at 1.0 mL/min., room temperature, Waters 990+ diode array detection) and found to contain 6

mcg/mL Compound I, $t_r = 15.8$ minutes.

EXAMPLE 3

Preparation of Compound I

A. Culturing Fungus MF5655

Culture MF5655 was inoculated into KF seed medium using one glass scoop of hyphal fragments preserved in sterile soil. The KF seed flask was incubated for 70 hours at 25° C, 220 rpm, 85% humidity. At the end of this incubation, 2.0 mL aliquots were aseptically transferred to each of 9 F202 solid production medium flasks and 9 CYS 80 liquid production medium flasks. These production flasks were then incubated at 25° C, 85% humidity, either statically (F202 flasks) or on a gyrotory shaker at 220 rpm (CYS 80 flasks) for 7 days. At harvest 45 mL of methanol were added to each F202 production flask and the solid growth was manually broken apart into smaller pieces. Solvent treated flasks were returned to the gyrotory shaker for agitation at 220 rpm for 30 minutes in order to further break apart the mycelial mass as well as to improve contact of the solvent with the cells. After shaking, the contents of these flasks were pooled by pouring the entire contents of the flasks (solids and all) into a 1 L flask. CYS 80 flasks were harvested by homogenizing the mycelial growth for 20 seconds at high speed using a Biohomogenizer/mixer (Biospec Products Inc., Bartlesville, OK). The whole broth contents of the CYS 80 flasks were then pooled into a 1 L Erlenmeyer flask.

B. Isolation of Compound I

A 400 mL portion of whole broth from the harvested liquid fermentation of Example 3, Part A was treated with 500 mL of 2-butanone and the mixture was stirred vigorously for two hours. The upper organic layer was removed by centrifugation and the lower aqueous layer was extracted a second time with 400 mL of 2-butanone. The first and the second 2-butanone extracts were combined and concentrated to an aqueous solution under reduced pressure. The concentrate was diluted with water to a volume of 100 mL and the pH of the mixture was adjusted to 2.0 with dilute hydrochloric acid. The aqueous mixture was then extracted twice with 100 mL of ethyl acetate. The combined ethyl acetate extract concentrated to a residue, which was reconstituted in 100 mL of a solution of 20 mM sodium formate in 6/4 acetonitrile-water. The pH of the solution was adjusted to 4.5.

The aqueous acetonitrile solution was adsorbed at 4 mL/min., onto 10 mL of BioRad AG4x4 resin (200-400 mesh), on the formate cycle at pH 4.5. The resin was washed with 150 mL of a pH 4.5 solution of 60 mM sodium formate in 6/4 acetonitrile-water. Compound I was eluted from the resin with a solution of pH 1.3 0.2 N $H_2SO_4$ in 6/4 acetonitrile-water. Eluate was collected in 10 mL fractions that were analyzed by HPLC for Compound I. Fractions containing I were combined, diluted with one half volume of 0.1 N HCl in 10% aqueous sodium chloride solution, and extracted with one volume of methylene chloride. The methylene chloride extract was dried with anhydrous sodium sulfate and evaporated to a Compound I containing residue. Reverse phase HPLC of the extract afforded an essentially pure sample of I.

EXAMPLE 4

Preparation of Compound I

A. Culturing Fungus MF5656

Culture MF5656 was inoculated into KF seed medium using one glass scoop of hyphal fragments preserved in sterile soil. The KF seed flask was incubated for 70 hours at 25° C, 220 rpm, 85% humidity. At the end of this incubation, 2.0 mL aliquots were aseptically transferred to each of 15 CYS 80 liquid production medium flasks. These production flasks were then incubated at 25° C, 220 rpm, 85% humidity for 7 days. Flasks were harvested by homogenizing the mycelial growth for 20 seconds at high speed using Biohomogneizer/mixer (Biospec Products Inc., Bartlesville, OK). The whole broth contents of the flasks were then pooled into a 1 L Erlenmeyer flask.

B. Isolation of Compound I

A 400 mL of whole broth from the harvested liquid fermentation of Example 4, Part A was treated with 500 mL of 2-butanone and the mixture was stirred vigorously for two hours. The upper organic layer was removed by centrifugation and the lower aqueous layer was extracted a second time with 400 mL of 2-butanone. The first and the second 2-butanone extracts were combined and concentrated to an aqueous solution under reduced pressure. The concentrate was diluted with water to a volume of 100 mL and the pH of the mixture was adjusted to 2.0 with dilute hydrochloric acid. The aqueous mixture was then extracted twice with 100 mL of ethyl acetate. The combined ethyl acetate extract were concentrated to a residue, which was reconstituted in 100 mL of a solution of 20 mM sodium formate in 6/4 acetonitrile-water. The pH of the solution was adjusted to 4.5.

The aqueous acetonitrile solution was adsorbed at 4 mL/min., onto 10 mL of BioRad AG4x4 resin (200-400 mesh), on the formate cycle at pH 4.5. The resin was washed with 150 mL of a pH 4.5 soltuion of 60 mM sodium formate in 6/4 acetonitrile-water. Compound I was eluted from the resin with a solution of pH 1.3 0.2N $H_2SO_4$ in 6/4 acetonitrile-water. Eluate was collected in 10 mL fractions that were analyzed by HPLC for Component I. Fractions containing I were combined, diluted with one half volume of 0.1 N HCl in 10% aqueous sodium chloride solution, and extracted with one volume of methylene chloride. The methylene chloride extract was dried with anhydrous sodium sulfate and evaporated to a Component I containing residue. Reverse phase HPLC of the extract afforded an essentially pure sample of I.

## EXAMPLE 5

### Preparation of Compound I

#### A. Culturing Fungus MF5657

Culture MF5657 was inoculated into KF seed medium using one glass scoop of the hyphal fragments preserved in sterile soil. The KF seed flask was incubated for 70 hours at 25° C, 220 rpm, 85% humidity. At the end of this incubation, 2.0 mL aliquots were aseptically transferred to each of 9 F202 solid production medium flasks and 10 CYS 80 liquid production medium flasks. These production flasks were then incubated at 25° C, 85% humidity, either statically (F202 flasks)) or on a gyrotory shaker at 220 rpm (CYS 80 flasks) for 14 days. At harvest 50 mL of methyl ethyl ketone were added to each F202 production flask and the solid growth was manually broken apart into smaller pieces. Solvent treated flasks were returned to the gyrotory shaker for agitation at 220 rpm for 30 minutes in order to further break apart the mycelial mass as well as to improve contact of the solvent with the cells. After shaking, the contents of these flasks were pooled by pouring the entire contents of the flasks (solids and all) into a 1 L flask. CYS 80 flasks were harvested by homogenizing the mycelial growth for 20 seconds at high speed using a Biohomogenizer/mixer (Biospec Products Inc., Bartlesville, OK). The whole broth contents of the CYS 80 flasks were then pooled into a 1 L Erlenmeyer flask.

#### B. Isolation of Compound I

A 400 mL of whole broth from the harvested liquid fermentation of Example 5, Part A was treated with 500 mL of 2-butanone and the mixture was stirred vigorously for two hours. The upper organic layer was removed by centrifugation and the lower aqueous layer was extracted a second time with 400 mL of 2-butanone. The first and the second 2-butanone extracts were combined and concentrated to an aqueous solution under reduced pressure. The concentrate was diluted with water to a volume of 100 mL and the pH of the mixture was adjusted to 2.0 with dilute hydrochloric acid. The aqueous mixture was then extracted twice with 100 mL of ethyl acetate. The combined ethyl acetate extracts were concentrated to a residue, which was reconstituted in 100 mL of a solution of 20 mM sodium formate in 6/4 acetonitrile-water. The pH of the solution was adjusted to 4.5.

The aqueous acetonitrile solution was adsorbed at 4 mL/min., onto 10 mL of BioRad AG4x4 resin (200-400 mesh), on the formate cycle at pH 4.5. The resin was washed with 150 mL of a pH 4.5 solution of 60 mM sodium formate in 6/4 acetonitrile-water. Compound I was eluted from the resin with a solution of pH 1.3 0.2N $H_2SO_4$ in 6/4 acetonitrile-water. Eluate was collected in 10 mL fractions that were analyzed by HPLC for Compound I. Fractions containing I were combined, diluted with one half volume of 0.1 N HCl in 10% aqueous sodium chloride solution, and extracted with one volume of methylene chloride. The methylene chloride extract was dried with anhydrous sodium sulfate and evaporated to a Compound I containing residue. Reverse phase HPLC of the extract afforded an essentially pure sample of I.

EXAMPLE 6

Preparation of Compound I

A. Culturing Fungus MF5663

Culture MF5663 was inoculated into KF seed medium using one glass scoop of hyphal fragments preserved in sterile soil. The KF seed flask was incubated for 72 hours at 25°C, 220 rpm, 85% humidity. At the end of this incubation, 2.0 mL aliquots were aseptically transferred to each of 15 CYS 80 liquid production medium flasks. These production flasks were then incubated at 25°C, 220 rpm, 85% humidity for 22 days. Flasks were harvested by homogenizing the mycelial growth for 20 seconds at high speed using a Biohomogenizer/mixer (Biospec Products Inc., Bartlesville, OK). The whole broth contents of the flasks were then pooled into a 1 L Erlenmeyer flask.

B. Isolation of Compound I

A 400 mL portion of whole broth from the harvested liquid fermentation of Example 6, Part A was treated with 500 mL of 2-butanone and the mixture was stirred vigorously for two hours. The upper organic layer was removed by centrifugation and the lower aqueous layer was extracted a second time with 400 mL of 2-butanone. The first and the second 2-butanone extracts were combined and concentrated to an aqueous solution under reduced pressure. The concentrate was diluted with water to a volume of 100 mL and the pH of the mixture was adjusted to 2.0 with dilute hydrochloric acid. The aqueous mixture was then extracted twice with 100 mL of ethyl acetate. The combined ethyl acetate extract were concentrated to a residue, which was reconstituted in 100 mL of a solution of 20 mM sodium formate in 6/4 acetonitrile-water. The pH of the solution was adjusted to 4.5.

The aqueous acetonitrile solution was adsorbed at 4 mL/min., onto 10 mL of BioRad4x4 resin (200-400 mesh), on the formate cycle at pH 4.5. The resin was washed with 150 mL of a pH 4.5 solution of 60 mM sodium formate in 6/4 acetonitrile-water. Compound I was eluted from the resin with a solution of pH 1.3 0.2N $H_2SO_4$ in 6/4 acetonitrile-water. Eluate was collected in 10 mL fractions that were analyzed by HPLC for Compound I. Fractions containing I were combined, diluted with one half volume of 0.1 N HCl in 10% aqueous sodium chloride solution, and extracted with one volume of methylene chloride. The methylene chloride extract was dried with anhydrous sodium sulfate and evaporated to a Compound I containing residue. Reverse phase HPLC of the extract afforded an essentially pure sample of I.

C. Physical Properties of Compound I

This compound has the molecular weight 730 by FAB-MS (observed $[M+H]^+$ at m/z 731, and with addition of lithium acetate $[M \cdot Li_3+Li]^+$ (ie., the lithium adduct of the trilithium salt at m/z 755). The molecular formula $C_{39}H_{54}O_{13}$ was determined by HR-EI measurement of the hexa-trimethylsilyl derivative (calc for $C_{39}H_{54}O_{13}+(SiC_3H_8)_6$-$CH_3$ 1147.5701, found 1147.5751).

$^1H$ NMR spectrum (400 MHz) ($CD_3OD$, 22°C): See figure 1.

$^{13}C$ NMR Chemical Shifts ($CD_3OD$, 22°C): 14.8, 15.2, 18.1, 25.3, 27.5, 30.1, 30.3 (2x), 33.2, 33.5 (2x), 33.9, 34.8, 36.7, 36.9, 38.6, 75.7, 76.7, 78.4, 81.0, 82.0, 91,1, 107.5, 125.7, 127.0 (2x), 127.9, 129.5(2x), 130.2, 131.0, 131.9, 132.57, 132.63, 139.3, 168.6, 170.3, 172.6, 173.6 ppm.

UV (MeOH): $\lambda$max is 250 nm ($\epsilon$ 23,000)

IR (as free acid; film on ZnSe): 3220 br, 2924, 2855, 2489 br, 1741, 1436, 1417, 1266, 1152, 1122, 1004, 967, 950, 804, 744, 695 $cm^{-1}$.

**Claims**

1. A biologically pure culture of Sporormiella intermedia, except strains ATCC 20985 and ATCC 20989, capable of producing a fermentation broth that inhibits squalene synthetase activity and which produces:

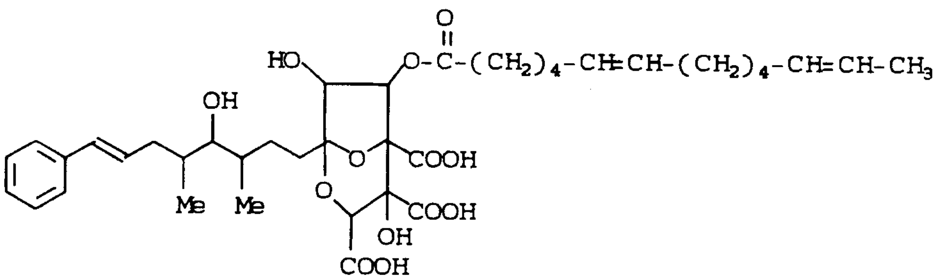

(I)

in recoverable amounts.

2. A culture of Sporormiella intermedia, except for strains ATCC 20985 and ATCC 20989, capable of producing a fermentation broth that inhibits squalene synthetase activity and which produces:

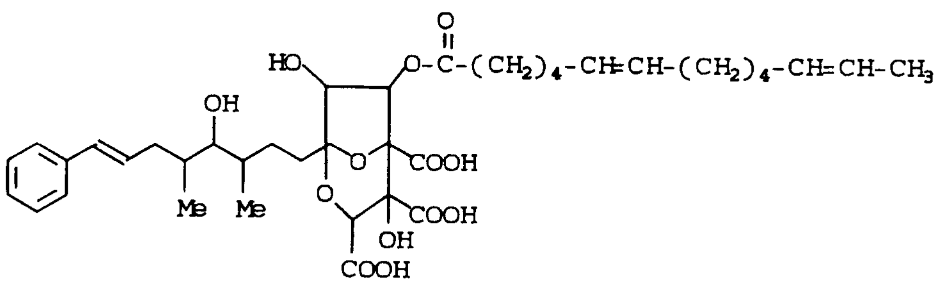

(I)

in recoverable amounts.

3. A biologically pure culture of Claim 1 selected from the group consisting of:
(a) MF5651 (ATCC 74072),
(b) MF5652 (ATCC 74073),
(c) MF5655 (ATCC 74074),
(d) MF5656 (ATCC 74075),
(e) MF5657 (ATCC 74076),
(f) MF5663 (ATCC 74083)
or an active mutant thereof.

4. A culture of Claim 2 selected from the group consisting of:
(a) MF5651 (ATCC 74072),
(b) MF5652 (ATCC 74073),
(c) MF5655 (ATCC 74074),
(d) MF5656 (ATCC 74075),
(e) MF5657 (ATCC 74076),
(f) MF5663 (ATCC 74083)
or an active mutant thereof.

5. A process of making a compound of structure:

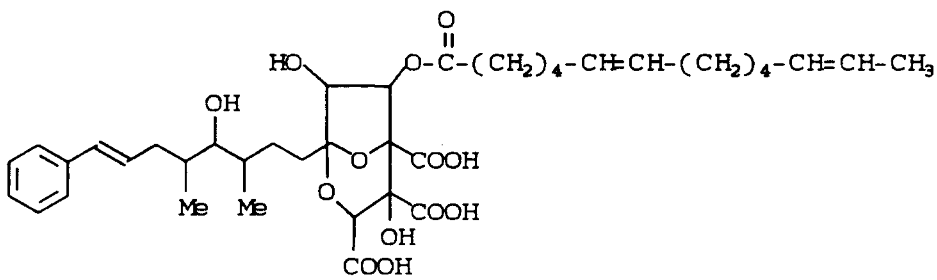

(I)

comprising cultivating a strain of Sporormiella intermedia, except strains ATCC 20985 and ATCC 20989, under conditions suitable for the formation of the compound and recovery of the compound.

**6.** A process of Claim 5 wherein the strain of Sporormiella intermedia is selected from the group consisting of:
(a) MF5651 (ATCC 74072),
(b) MF5652 (ATCC 74073),
(c) MF5655 (ATCC 74074),
(d) MF5656 (ATCC 74075),
(e) MF5657 (ATCC 74076),
(f) MF5663 (ATCC 74083)
or an active mutant thereof.

**7.** A process of Claim 5 wherein the cultivation is carried out for 7 to 21 days.

**8.** A process of Claim 6 wherein the cultivation is carried out for 7 to 21 days.

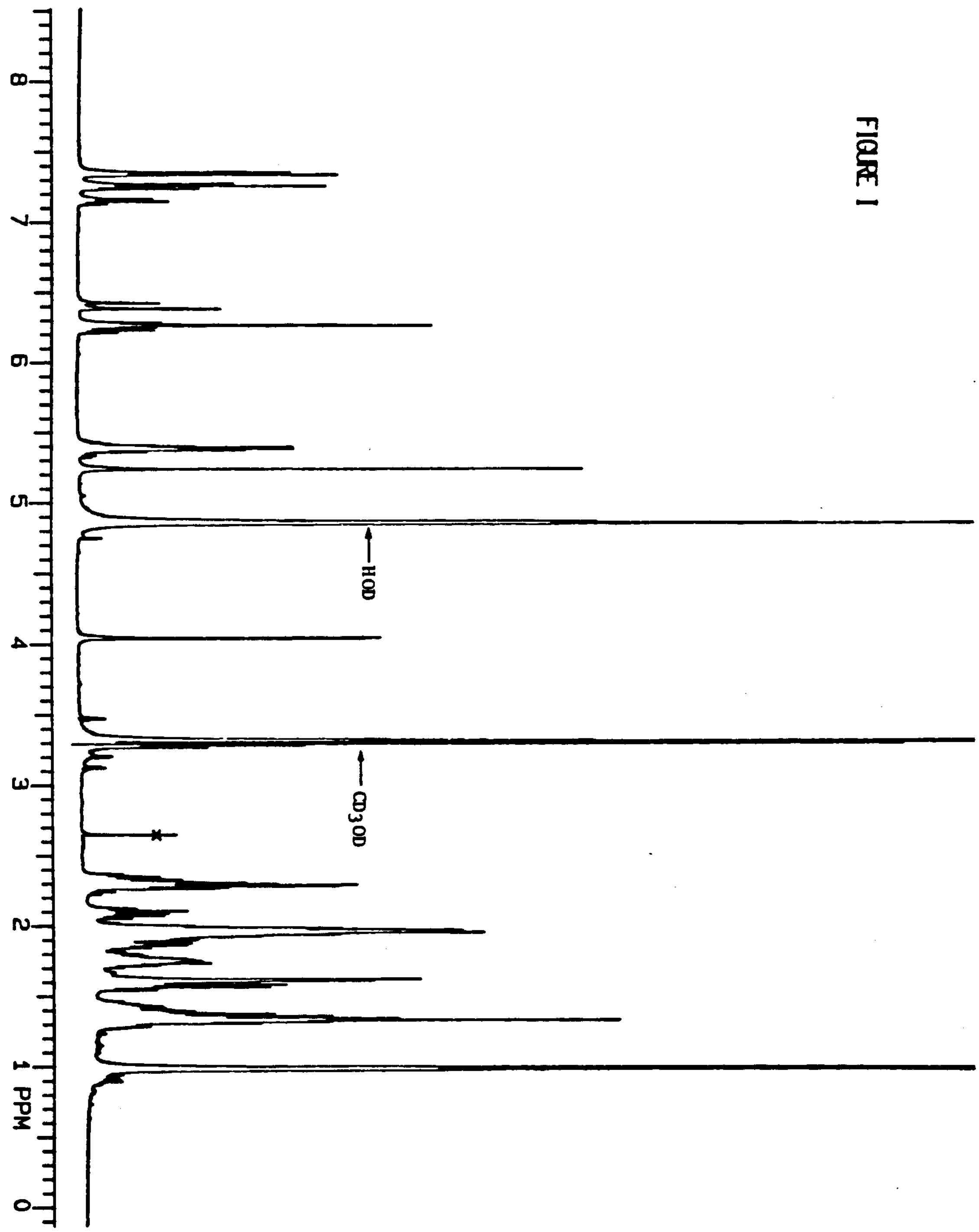


FIGURE I

European Patent Office

# EUROPEAN SEARCH REPORT

Application Number

EP 92 20 1829

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-5 026 554 (K.F.BARTIZAL ET AL.)<br>* the whole document *<br>--- | 1-8 | C12N1/14<br>C12P7/40<br>//(C12P7/40, C12R1/645) |
| A | DATABASE WPIL<br>Week 8918,<br>Derwent Publications Ltd., London, GB;<br>AN 89-136261<br>& JP-A-1 083 078 (TOYO JOZO KK) 1989<br>* abstract *<br>--- | 1-8 | |
| P,X | EP-A-0 448 393 (MERCK & CO. INC.)<br>* the whole document *<br>----- | 1-8 | |
| | | | TECHNICAL FIELDS SEARCHED (Int. Cl.5) |
| | | | C12P |

The present search report has been drawn up for all claims

| Place of search | Date of completion of the search | Examiner |
|---|---|---|
| BERLIN | 23 OCTOBER 1992 | GURDJIAN D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document